# EUROPEAN PATENT APPLICATION

(11) **EP 4 166 655 A1**
(43) Date of publication of application: **19.04.2023**
(21) Application number: 21855587.8
(22) Date of filing: 12.08.2021
(51) Int. Cl.: C12N 5/10, C12N 15/12, C12N 15/24, C12N 15/62, C12N 15/867, A61K 39/00, A61P 35/00, A61P 31/00, A61P 33/00

(54) **ENGINEERED IMMUNE CELL AND USE THEREOF**

(30) Priority: 13.08.2020 CN 202010813295
(71) Applicant: Nanjing Bioheng Biotech Co., Ltd, Nanjing, Jiangsu 210061 (CN)
(72) Inventor: XING, Yun, Nanjing Jiangsu 210061 (CN); REN, Jiangtao, Nanjing Jiangsu 210061 (CN); HE, Xiaohong, Nanjing Jiangsu 210061 (CN); WANG, Yanbin, Nanjing Jiangsu 210061 (CN); HAN, Lu, Nanjing Jiangsu 210061 (CN)
(74) Representative: Rimini, Rebecca
(86) International application number: PCT/CN2021/112174
(87) International publication number: WO 2022/033537

(57) **Abstract**

Provided is an engineered immune cell. The engineered immune cell expresses (i) a chimeric receptor, and (ii) exogenous CCL3, CCL4 and/or CCL5, has improved tumor killing activity, and can be used to treat cancer, infection or autoimmune diseases.

## Description

### Technical Field

The present disclosure belongs to the field of immunotherapy. More specifically, the present disclosure relates to an engineered immune cell, which expresses (i) a chimeric receptor, and (ii) exogenous CCL3, CCL4 and/or CCL5. More preferably, the chimeric receptor is a chimeric antigen receptor or a T cell receptor.

### Background Art

Tumor immunotherapy mainly eliminates tumor cells by regulating the human immune system and tumor microenvironment, and finally relying on autoimmunity. The immune system is a unified whole, and innate immunity also plays a quite important role in tumor immunity.

Some antigen presenting cells, such as dendritic cells and macrophages, are bridges connecting innate immunity and acquired immunity. The antigen presenting cells can recognize a tumor antigen and present the same to the acquired immune system, and activate tumor specific T cells, thereby eliminating the tumor. Therefore, increasing the tumor killing effect of the immune system by enhancing the antigen presentation process is an important research direction of tumor immunity.

CAR cell therapy is an important tumor cell immunotherapy. Successful control of tumor by CAR cell generally requires following several processes: immune system activation, activation and amplification of CAR cell, and infiltration of activated CAR cell into the tumor tissue to kill the tumor cell. However, there is a general problem with the current CAR cell therapy, i.e., the tumor microenvironment has an inhibitory effect on the CAR cell, such that the CAR cell cannot infiltrate the tumor tissue. Therefore, how to reduce the inhibitory effect of the tumor microenvironment on CAR cell, improve survival time of CAR cell, or recruit other immune cells to act synergistically with CAR cell is quite important for improving the therapeutic effect of CAR cell.

Conventional type 1 dendritic cell (conventional DC1, cDC 1) is a subset of dendritic cells, and is a main immune cell presenting the tumor antigens. Research results show that cDC1 can effectively present tumor-associated antigens, particularly necrotic cell-associated antigens, effectively induce antigen-specific CD8+ T cell response, and play an extremely important role in the *in-vivo* tumor killing process. Both mouse and human studies show that the distribution of cDC1 in the tumor microenvironment is positively correlated with the anti-tumor immune response, and thus is an important evaluation parameter of tumor-related immune score. The cDC1 is less distributed in mice and humans, and is almost invisible in the tumor microenvironment of mouse and human with a low tumor immune response rate. Optimizing the role of cDC1 in tumor therapy is an important direction of research for improving the tumor immunotherapy effect.

Inflammatory chemokines play important roles in recruiting relevant immune cells into the tumor microenvironment, driving cellular interactions and molecular signaling cascades and the like, thus determining the ultimate outcome of the host antitumor immune response. Studies have shown that the macrophage inflammatory protein (MIP)-1 family and related proteins, composed of CCL3 (MIP-1a), CCL4 (MIP-1b), and CCL5 (RANTES), may be a major determinant of immune cell infiltration in certain tumors by directly recruiting antigen-presenting cells (such as recruiting dendritic cells to the tumor site). In addition, it has been reported that MIP-1 family-related proteins enriched in tumor sites may recruit natural killer cells (NK) to tumor sites, thereby promoting the accumulation of CD103⁺ cDC in tumor sites, and increasing the production of chemokines CXCL9 and CXCL10, effectively inhibiting tumor progression.

Therefore, there is a need for a new immunotherapeutic approach that can effectively differentiate or recruit cDC1 dendritic cells, so as to improve tumor antigen presentation efficiency, induce the body's adoptive immune response, thus improving the efficacy of CAR cell therapy.

### Summary

In a first aspect, the present disclosure provides a novel engineered immune cell, expressing (i) a chimeric receptor, and (ii) an exogenous CCL3, CCL4 and/or CCL5 gene.

In an embodiment, the CCL3 gene has at least 90% identity to a nucleic acid sequence represented by SEQ ID NO: 77 or 79, or a polypeptide encoded by the CCL3 gene has at least 90% identity to an amino acid sequence represented by SEQ ID NO: 78 or 80.

In an embodiment, the CCL4 gene has at least 90% identity to a nucleic acid sequence represented by SEQ ID NO: 81 or 83, or a polypeptide encoded by the CCL4 gene has at least 90% identity to an amino acid sequence represented by SEQ ID NO: 82 or 84.

In an embodiment, the CCL5 gene has at least 90% identity to a nucleic acid sequence represented by SEQ ID NO: 85 or 87, or a polypeptide encoded by the CCL5 gene has at least 90% identity to an amino acid sequence represented by SEQ ID NO: 86 or 88.

In an embodiment, the engineered immune cell further expresses (iii) an exogenous interleukin.

In an embodiment, the interleukin is IL-2, IL-7, IL-12, IL-15, IL-21, IL-17, IL-18, IL-23, IL-33, or a subunit thereof, or a combination thereof, or a combination of subunits thereof, preferably IL-7.

In an embodiment, an encoding gene of the interleukin has at least 90% identity to a nucleic acid sequence represented by SEQ ID NO: 41, 43, 45, 47, 49, 51, 53, 55, 57, 59, 61, 63, 65, 67, 69, 71, 73, 75, or the interleukin has at least 90% identity to an amino acid sequence represented by SEQ ID NO: 42, 44, 46, 48, 50, 52, 54, 56, 58, 60, 62, 64, 66, 68, 70, 72, 74, 76.

In an embodiment, the interleukin, CCL4 or CCL5 expressed by the engineered immune cell is a fusion protein or mutant that is resistant to protease hydrolysis.

In an embodiment, the chimeric receptor is a chimeric antigen receptor or a T cell receptor. Preferably, the chimeric receptor is a chimeric antigen receptor, which comprises a ligand binding domain, a transmembrane domain, a co-stimulatory domain, and an intracellular signaling domain. In the above, the ligand binding domain may be selected from the group consisting of an immunoglobulin molecule, Fab, Fab', F(ab')2, Fv fragment, scFv, disulfide bond-linked Fv (sdFv), heavy chain variable region (VH) or light chain variable region (VL) of an antibody, Fd fragment consisting of VH and CH1 domains, linear antibody, single domain antibody, nanobody, and non-immunoglobulin antigen binding scaffold.

In an embodiment, the chimeric receptor binds to a target selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-11Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D, and any combination thereof. Preferably, the target is selected from the group consisting of: CD19, CD20, CD22, CD30, CD33, CD38, CD123, CD138, CD171, MUC1, AFP, Folate receptor α, CEA, PSCA, PSMA, Her2, EGFR, IL13Ra2, GD2, NKG2D, EGFRvIII, CS1, BCMA, mesothelin, and any combination thereof.

In an embodiment, the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137, and CD154. Preferably, the transmembrane domain is selected from a transmembrane domain of CD8 α, CD4, CD28, and CD278.

In an embodiment, the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d. Preferably, the intracellular signaling domain is a signaling domain containing CD3 ζ.

In an embodiment, the co-stimulatory domain is one or more co-stimulatory signaling domains of a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, CD94, LTB, ZAP70, and a combination thereof. Preferably, the co-stimulatory domain is a co-stimulatory signaling domain of CD27, CD28, CD134, CD137 or CD278 or a combination thereof.

In an embodiment, the expression or activity of the exogenous interleukin, CCL3, CCL4, and/or CCL5 is constitutive expression. In another embodiment, the expression or activity of the exogenous interleukin, CCL3, CCL4, and/or CCL5 is conditional expression. For example, the conditional expression is achieved by operably linking the exogenous genes to an inducible, repressible or tissue-specific promoter.

In an embodiment, the interleukin, CCL3, CCL4, and/or CCL5 may be operably linked to a localization domain, wherein the localization domain can locate the exogenous gene of the present disclosure at a specific cellular position for expression, for example, a cell membrane, a specific organelle in the cytoplasm, e.g., endoplasmic reticulum, golgi apparatus, nucleus, etc. The localization domain includes, but is not limited to, a nuclear localization signal, a leader peptide, a transmembrane domain, and the like. In an embodiment, the exogenous genes, i.e., interleukin, CCL3, CCL4, and/or CCL5 of the present disclosure are operably linked to the transmembrane domain, so as to be anchored on the surface of the engineered immune cell to be expressed.

In an embodiment, the immune cell is selected from the group consisting of a T cell, a macrophage, a dendritic cell, a monocyte, an NK cell, or an NKT cell. Preferably, the T cell is a CD4+/CD8+ T cell, a CD4+ helper T cell, a CD8+ T cell, a tumor infiltrating cell, a memory T cell, a naive T cell, a γδ-T cell, or a αβ-T cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell or a hematopoietic stem cell.

In a second aspect, the present disclosure provides a nucleic acid molecule, comprising (i) a nucleic acid sequence encoding a chimeric receptor, and (ii) a nucleic acid sequence encoding CCL3, CCL4 and/or CCL5. Preferably, the chimeric receptor is a chimeric antigen receptor or a T cell receptor, more preferably a chimeric antigen receptor.

In an embodiment, the nucleic acid molecule further comprises a nucleic acid sequence encoding an interleukin. Preferably, the interleukin is IL-2, IL-7, IL-12, IL-15, IL-21, IL-17, IL-18, IL-23, IL-33, or a subunit thereof, or a combination thereof, or a combination of subunits thereof, more preferably IL-7, a subunit thereof, or a combination of subunits thereof. Preferably, the nucleic acid is DNA or RNA.

The present disclosure further provides a vector comprising the above nucleic acid molecule. Specifically, the vector is selected from the group consisting of plasmid, retrovirus, lentivirus, adenovirus, vaccinia virus, Rous Sarcoma Virus (RSV), polyoma virus, and adeno-associated virus (AAV). In some embodiments, the vector further comprises elements such as an origin autonomously replicating in an immune cell, a selectable marker, a restriction enzyme cleavage site, a promoter, a poly-A tail (polyA), 3' UTR, 5' UTR, an enhancer, a terminator, an insulator, an operon, a selectable marker, a reporter gene, a targeting sequence, and/or a protein purification tag. In a specific embodiment, the vector is an *in vitro* transcription vector.

In an embodiment, the present disclosure further provides a kit, which comprises the engineered immune cell, the nucleic acid molecule, or the vector of the present disclosure.

In an embodiment, the present disclosure further provides a pharmaceutical composition, which comprises the engineered immune cell, the nucleic acid molecule, or the vector of the present disclosure, and one or more pharmaceutically acceptable excipients.

In a third aspect, the present disclosure further provides a method of treating a subject with cancer, infection or autoimmune disease, including administering to the subject an effective amount of the immune cell, the nucleic acid molecule, the vector or the pharmaceutical composition according to the present disclosure.

In an embodiment, the cancer is a solid tumor or a hematologic tumor. More specifically, the cancer is selected from the group consisting of: brain glioma, blastoma, sarcoma, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (e.g., small cell lung cancer, non-small cell lung cancer, lung adenocarcinoma and squamous lung cancer), melanoma, myeloma, neuroblastoma, oral cancer (e.g., lips, tongue, mouth, and pharynx), ovarian cancer, pancreatic cancer, prostate cancer, mesothelioma, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer, Waldenstrom macroglobulinemia, lymphoma (including Hodgkin's lymphoma and non-Hodgkin's lymphoma, such as B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, Burkitt lymphoma, diffuse large B cell lymphoma, follicular lymphoma, MALT lymphoma, marginal zone lymphoma, plasmablastic lymphoma, plasmacytoid dendritic cell tumor), leukemia (including acute leukemia such as acute lymphoblastic leukemia, acute myelogenous leukemia, acute nonlymphocytic leukemia such as acute myeloblastic leukemia (including undifferentiated and partially differentiated), acute promyelocytic leukemia, acute myelomonocytic leukemia, acute monocytic leukemia, erythroleukemia, acute megakaryoblastic leukemia; chronic leukemia such as chronic myelogenous leukemia, chronic lymphocytic leukemia, chronic monocytic leukemia; and other special types of leukemia such as hairy cell leukemia, prolymphocytic leukemia, plasma cell leukemia, adult T-cell leukemia, eosinophilic leukemia, basophilic leukemia, etc.), blastic plasmacytoid dendritic cell tumor, malignant lymphoproliferative disease, myeloid dysplasia, multiple myeloma, myelodysplasia, and post-transplant lymphoproliferative disorder (PTLD).

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

The advantages of the engineered immune cell of the present disclosure lie in that the co-expressed CCL3, CCL4 and/or CCL5 and optionally an interleukin such as IL7 can effectively recruit DC cells to the tumor site, and increase the proliferation and survival period of engineered immune cell, thus, reducing the inhibitory effect of the tumor microenvironment on the engineered immune cell, and improving the tumor killing ability of the engineered immune cell. Moreover, the recruited DC cells can activate the adoptive immune recognition of the body's own T cells, which forms synergistic effect with the engineered immune cell. Eventually the inhibition for tumor is enhanced.

### Brief Description of Drawings

FIG. 1: CD19 expression rate of Panc02-mCD19 cells.
FIG. 2: CAR expression levels of CAR-T cells detected by flow cytometry.
FIG. 3: IL-7 expression levels of CAR-T cells detected by ELISA.
FIG. 4: CCL4 expression levels of CAR-T cells detected by ELISA.
FIG. 5: CCL5 expression levels of CAR-T cells detected by ELISA.
FIG. 6: IFN-γ release levels after co-culture of CAR-T cells with target cells and non-target cells, respectively.
FIG. 7: Curves of body weight change of mice with pancreatic cancer after treated with CAR-T cells expressing CCL4 or CCL5.
FIG. 8: Curves of tumor growth of mice with pancreatic cancer after treated with CAR-T cells expressing CCL4 or CCL5.
FIG. 9: Curves of body weight change of mice with pancreatic cancer after treated with CAR-T cells expressing CCL3.
FIG. 10: Curves of tumor growth of mice with pancreatic cancer after treated with CAR-T cells expressing CCL3.

### Detailed Description of Embodiments

Unless otherwise indicated, all scientific and technical terms used herein have the same meaning as commonly understood by one of ordinary skill in the art to which the present disclosure belongs.

### Chimeric receptor

As used herein, the term "chimeric receptor" refers to a molecule expressed on a cell surface and being capable of specifically binding to a target molecule (e.g., ligand). Such surface molecule generally comprises a ligand binding domain capable of specifically binding to a ligand, a transmembrane domain anchoring the surface molecule to the cell surface, and an intracellular domain responsible for signaling. Examples of such chimeric receptor include, for example, T cell receptor or chimeric antigen receptor.

As used herein, the term "T cell receptor" or "TCR", refers to a membrane protein complex that responds to antigen presentation and participates in T cell activation. Stimulation of TCR is triggered by major histocompatibility complex molecules (MHC) on antigen presenting cells, which present antigen peptides to T cells and bind the same to TCR complexes so as to induce a series of intracellular signaling. TCR is composed of six peptide chains forming heterodimers respectively, and is generally sorted into αβ type and γδ type. Each peptide chain includes a constant region and a variable region, wherein the variable region is responsible for binding to specific antigens and MHC molecules. The variable region of TCR may comprise a ligand binding domain or is operably linked to a ligand binding domain, wherein the ligand binding domain is defined as follows.

As used herein, the term "chimeric antigen receptor" or "CAR" refers to an artificially constructed hybrid polypeptide, where the hybrid polypeptide generally includes a ligand binding domain (e.g., an antibody or antibody fragment), a transmembrane domain, a co-stimulatory domain, and an intracellular signaling domain, and various domains are linked via a linker. CAR can redirect the specificity and reactivity of T cell and other immune cells to a selected target in a non-MHC-restricted manner by utilizing the antigen binding properties of monoclonal antibodies. Non-MHC-restricted antigen recognition gives CAR cell the ability to recognize an antigen independent of antigen processing, thus bypassing the major mechanism of tumor escape. Besides, when expressed within T cells, CAR advantageously does not dimerize with α chain and β chain of the endogenous T cell receptor (TCR).

As used herein, "ligand binding domain" refers to any structure or functional variant thereof that can bind to a ligand (e.g. antigen). The ligand binding domain may be an antibody structure, including, but not limited to, monoclonal antibody, polyclonal antibody, recombinant antibody, human antibody, humanized antibody, murine antibody, chimeric antibody, and functional fragment thereof. Examples of ligand binding domains include, but are not limited to, an immunoglobulin molecule, Fab, Fab', F(ab')2, Fv fragment, scFv, disulfide bond-linked Fv (sdFv), heavy chain variable region (VH) or light chain variable region (VL) of an antibody, Fd fragment consisting of VH and CH1 domains, linear antibody, single domain antibody, nanobody, and non-immunoglobulin antigen binding scaffold, such as, recombinant fibronectin domain, DARPIN, affibody, affilin, adnectin, affitin, obodies, repebody, fynomer, alpha body, avimer, atrimer, centyrin, pronectin, anticalin, kunitz-type domain, Armadillo repeat protein, etc. Preferably, the ligand binding domain is selected from the group consisting of Fab, scFv, single domain antibody, nanobody, or functional fragment thereof. In the present disclosure, the ligand binding domain may be monovalent or bivalent, and may be a monospecific, bispecific or multispecific antibody comprising one or more ligand binding domains.

"Fab" refers to any one of two identical fragments produced after an immunoglobulin molecule is cleaved by papain, and consists of an intact light chain and a heavy chain N-terminal part linked by a disulfide bond, wherein the heavy chain N-terminal part includes a heavy chain variable region and CH1. Compared with intact IgG, Fab has no Fc fragment, has relatively high fluidity and tissue penetration ability, and can univalently bind to an antigen without mediating antibody effects.

"Single chain antibody" or "scFv" is an antibody composed of antibody's heavy chain variable region (VH) and light chain variable region (VL) linked by a linker. The optimal length and/or amino acid composition of the linker can be selected. The length of the linker will significantly affect the variable region folding and interaction of the scFv. In fact, intrachain folding can be prevented if a shorter linker (e.g. 5-10 amino acids) is used. Regarding the selection of size and composition of the linker, see, e.g., Hollinger et al., 1993 Proc Natl Acad. Sci. U.S.A. 90:6444-6448; US patent applications with publication Nos. 2005/0100543, 2005/0175606, 2007/0014794; and PCT applications with publication Nos. WO2006/020258 and WO2007/024715, which are incorporated herein by reference in their entirety. The scFv may contain a VH and a VL linked in any order, e.g., VH-linker-VL or VL-linker-VH.

"Single domain antibody" or "sdAb" refers to an antibody that naturally lacks light chain, and the antibody contains only one heavy chain variable region (VHH) and two conventional CH2 and CH3 regions, also known as "heavy chain antibody".

"Nanobody" or "Nb" refers to a VHH structure that is individually cloned and expressed, which has structural stability and binding activity to an antigen comparable to those of the original heavy chain antibody, and is the smallest unit currently known to be capable of binding to a target antigen.

The term "functional variant" or "functional fragment" refers to a variant that substantially includes the amino acid sequence of a parent, but, compared with the parent amino acid sequence, contains at least one amino acid modification (i.e., substitution, deletion, or insertion), provided that the variant retains the biological activity of the parent amino acid sequence. In an embodiment, the amino acid modification is preferably a conservative modification.

As used herein, the term "conservative modification" refers to amino acid modification that does not significantly affect or alter the binding characteristics of the antibody or antibody fragment containing the amino acid sequence. These conservative modifications include amino acid substitution, addition, and deletion. The modifications can be introduced into the chimeric antigen receptor of the present disclosure by standard techniques known in the art, such as site-directed mutagenesis and PCR-mediated mutagenesis. The conservative amino acid substitution is a substitution in which the amino acid residue is replaced with an amino acid residue having a similar side chain. Amino acid residue families having a similar side chain have been defined in the art, including basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine), and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with completely identical sequences have 100% identity. Those skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

The selection of ligand binding domain depends on the cell surface marker on a target cell to be recognized and associated with a specific disease state, for example, a tumor specific antigen or a tumor associated antigen. Thus, in an embodiment, the ligand binding domain of the present disclosure binds to one or more targets selected from the group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D, and any combination thereof. Preferably, the target is selected from the group consisting of: CD19, CD20, CD22, BAFF-R, CD33, EGFRvIII, BCMA, GPRC5D, PSMA, ROR1, FAP, ERBB2 (Her2/neu), MUC1, EGFR, CAlX, WT1, NY-ESO-1, CD79a, CD79b, GPC3, Claudin18.2, NKG2D, and any combination thereof. Depending on the antigen to be targeted, the CAR of the present disclosure may be designed to include a ligand binding domain specific for the antigen. For example, if CD19 is the antigen to be targeted, a CD19 antibody can be used as a ligand binding domain of the present disclosure.

In an embodiment, the chimeric antigen receptor of the present disclosure targets CD19, CD22, or a combination thereof. In a preferred embodiment, the chimeric antigen receptor of the present disclosure comprises an anti-CD19 antibody, which comprises a light chain variable region sequence having at least 70%, preferably at least 80%, more preferably at least 90%, 95%, 97%, 99% or 100% sequence identity to an amino acid sequence represented by positions 1-107 of SEQ ID NO: 1 or positions 1-107 of SEQ ID NO: 25, and a heavy chain variable region sequence having at least 70%, preferably at least 80%, more preferably at least 90%, 95%, 97%, 99% or 100% sequence identity to an amino acid sequence represented by positions 123-242 of SEQ ID NO: 1 or positions 123-238 of SEQ ID NO: 25. In a preferred embodiment, the chimeric antigen receptor of the present disclosure comprises an anti- CD22 antibody, which comprises a heavy chain variable region sequence having at least 70%, preferably at least 80%, more preferably at least 90%, 95%, 97%, 99% or 100% sequence identity to an amino acid sequence represented by positions 1-124 of SEQ ID NO: 27, and a light chain variable region sequence having at least 70%, preferably at least 80%, more preferably at least 90%, 95%, 97%, 99% or 100% sequence identity to an amino acid sequence represented by positions 143-249 of SEQ ID NO: 27.

As used herein, the term "transmembrane domain" refers to a polypeptide structure that enables expression of a chimeric antigen receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric antigen receptor binds to the target antigen. The transmembrane domains particularly suitable for use in the present disclosure may be derived from, for example, a TCR α chain, a TCR β chain, a TCR γ chain, a TCR δ chain, a CD3 ζ subunit, a CD3 ε subunit, a CD3 γ subunit, a CD3 δ subunit, CD45, CD4, CD5, CD8 α, CD9, CD16, CD22, CD33, CD28, CD37, CD64, CD80, CD86, CD134, CD137, CD154, and functional fragments thereof. Alternatively, the transmembrane domain may be synthesized and may mainly contain a hydrophobic residue such as leucine and valine. Preferably, the transmembrane domain is derived from a CD8 α chain or CD28, which has at least 70%, preferably at least 80%, more preferably at least 90%, 95%, 97% or 99% or 100% sequence identity to an amino acid sequence represented by SEQ ID NO: 3, 5 or 30, or an encoding sequence thereof has at least 70%, preferably at least 80%, more preferably at least 90%, 95%, 97% or 99% or 100% sequence identity to a nucleotide sequence represented by SEQ ID NO: 4, 6 or 29.

In an embodiment, the chimeric antigen receptor of the present disclosure further may contain a hinge region located between the ligand binding domain and the transmembrane domain. As used herein, the term "hinge region" generally refers to any oligopeptide or polypeptide that functions to link a transmembrane domain to a ligand binding domain. Specifically, the hinge region serves to provide greater flexibility and accessibility to the ligand binding domain. The hinge region may contain up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region contains a hinge region portion of a CD8 α chain, an Fc γ RIII α receptor, an IgG4, or an IgG1, more preferably a hinge from CD8 α, CD28 or IgG4, which has at least 70%, preferably at least 80%, more preferably at least 90%, 95%, 97% or 99% or 100% sequence identity to an amino acid sequence represented by SEQ ID NO: 19, 21, 23 or 36, or an encoding sequence thereof has at least 70%, preferably at least 80%, more preferably at least 90%, 95%, 97% or 99% or 100% sequence identity to a nucleotide sequence represented by SEQ ID NO: 20, 22, 24 or 35.

As used herein, the term "intracellular signaling domain" refers to a protein portion that transduces an effector function signal and guides a cell to perform a specified function. The intracellular signaling domain is responsible for intracellular primary signaling after the ligand binding domain binds to the antigen, thus causing activation of immune cell and immune reaction. In other words, the intracellular signaling domain is responsible for activating at least one of the normal effector functions of the immune cells in which the CAR is expressed. For example, the effector functions of T cell can be cytolytic activity or auxiliary activity, including secretion of cytokines.

In an embodiment, the intracellular signaling domain contained in the chimeric antigen receptor of the present disclosure may be cytoplasmic sequences of a T cell receptor and a co-receptor, upon antigen receptor binding, which act together to initiate primary signaling, as well as any derivative or variant of these sequences and any synthetic sequence having the same or similar function. The intracellular signaling domain may contain many immunoreceptor tyrosine-based activation motifs (ITAM). Non-limiting examples of intracellular signaling domain of the present disclosure include, but are not limited to, those derived from FcR γ, FcR β, CD3 γ, CD3 δ, CD3 ε, CD3 ζ, CD22, CD79a, CD79b, and CD66d. In a preferred embodiment, the signaling domain of the CAR of the present disclosure may contain a CD3 ζ signaling domain, and the signaling domain has at least 70%, preferably at least 80%, more preferably at least 90%, 95%, 97%, or 99% or 100% sequence identity to an amino acid sequence represented by SEQ ID NO: 11, 13 or 34, or an encoding sequence thereof has at least 70%, preferably at least 80%, more preferably at least 90%, 95%, 97%, or 99% or 100% sequence identity to a nucleotide sequence represented by SEQ ID NO: 12, 14 or 33.

In an embodiment, the chimeric antigen receptor of the present disclosure contains one or more co-stimulatory domains. The co-stimulatory domain may be an intracellular functional signaling domain from a co-stimulatory molecule, which contains an entire intracellular portion of the co-stimulatory molecule, or a functional fragment thereof. "Co-stimulatory molecule" refers to a homologous binding partner that specifically binds to a co-stimulatory ligand on a T cell, thereby mediating a co-stimulatory response (e.g. proliferation) of the T cell. The co-stimulatory molecule includes, but is not limited to, MHC class 1 molecules, BTLA, and Toll ligand receptors. Non-limiting examples of the co-stimulatory domain of the present disclosure include, but are not limited to, co-stimulatory signaling domains derived from a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA) , CD276 (B7-H3), CD278 (ICOS), CD357 (GITR), DAP10, DAP12, LAT, NKG2C, SLP76, PD-1, LIGHT, TRIM, CD94, LTB and ZAP70. Preferably, the co-stimulatory domain of the CAR of the present disclosure is from 4-1BB, CD28, CD27, OX40, or a combination thereof. In an embodiment, the co-stimulatory domain contained in the CAR of the present disclosure has at least 70%, preferably at least 80%, more preferably at least 90%, 95%, 97%, or 99% or 100% sequence identity to an amino acid sequence represented by SEQ ID NO: 7 or 9, or an encoding sequence of the co-stimulatory domain has at least 70%, preferably at least 80%, more preferably at least 90%, 95%, 97%, or 99% or 100% sequence identity to a nucleotide sequence represented by SEQ ID NO: 8 or 10.

In an embodiment, the CAR of the present disclosure further may contain a signal peptide such that when it is expressed in a cell such as a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present disclosure are well known to those skilled in the art, for example, signal peptides derived from CD8 α, IgG1, GM-CSFRα, B2M and so on. In an embodiment, the signal peptide that can be used in the present disclosure has at least 70%, preferably at least 80%, more preferably at least 90%, 95%, 97%, or 99% or 100% sequence identity to an amino acid sequence represented by SEQ ID NO: 15, 17 or 40, or an encoding sequence of the signal peptide has at least 70%, preferably at least 80%, more preferably at least 90%, 95%, 97%, or 99% or 100% sequence identity to a nucleotide sequence represented by SEQ ID NO: 16, 18 or 39.

In an embodiment, the CAR of the present disclosure further may contain a switch structure to regulate the expression time of the CAR. For example, the switch structure may be in a form of dimerization domain, which causes a conformational change by binding to a corresponding ligand thereof, and exposes the extracellular binding domain to enable its binding to a targeted antigen, thereby activating a signaling pathway. Alternatively, a switch domain also may be used to link the binding domain and signaling domain, respectively, and only when the switch domains are bound to each other (for example, in the presence of an inducing compound), the binding domain and the signaling domain can be linked together through a dimer, thereby activating signaling pathway. The switch structure also can be in the form of a masking peptide. The masking peptide can shield the extracellular binding domain, and prevent it from binding to the targeted antigen. When the masking peptide is cleaved by, for example, a protease, the extracellular binding domain is exposed, making it become a "normal" CAR structure. A variety of switch structures known to those skilled in the art can be used in the present disclosure.

In an embodiment, the CAR of the present disclosure further may contain a suicide gene, to make it express a cell death signal that can be induced by an exogenous substance, so as to eliminate the CAR cell when needed (e.g., when serious toxic side effects are produced). For example, the suicide gene may be in the form of an inserted epitope, e.g., a CD20 epitope, an RQR8, etc., and when needed, the CAR cell can be eliminated by adding an antibody or reagent that targets these epitopes. The suicide gene also may be herpes simplex virus thymidine kinase (HSV-TK), which gene can induce the cell to die when receiving ganciclovir treatment. The suicide gene further may be iCaspase-9, and dimerization of iCaspase-9 can be induced by a chemical induction drug such as AP1903 and AP20187, so as to activate the downstream Caspase3 molecule, and cause apoptosis. A variety of suicide genes known to those of skill in the art can be used in the present disclosure.

### CC chemokine

Chemokines can be divided into four subfamilies: CXC, CC, C, and CX3C, according to the arrangement of their N-terminal cysteines. Among them, the CC chemokine is named for the presence of two adjacent cysteine-cysteine (cys-cys or C-C) residues. Examples of CC chemokines are those that bind to CCR1 and CCR5, including MIP-1α, MIP-1β, CCL5, MCP-1, MCP-2, MCP-3, I-309, and the like.

Macrophage inflammatory protein (MIP) has two main forms: α (MIP-1α, also known as CCL3) and β (MIP-1β, also known as CCL4), which were first isolated from the culture fluid of macrophages activated by lipopolysaccharide. They both have similar sequences and activities, are mainly produced by macrophages, monocytes and dendritic cells, etc., and participate in the immune response to inflammation and infection by binding to the chemokine receptor 5 (CCR5) on the cell surface. Studies have shown that CCL3 and CCL4 participate in the host defense response of many infectious diseases such as tuberculosis, and mediate the directed migration of immune cells.

CCL5, also known as RANTES, is a small molecule protein with a molecular weight of 8000. The gene of human CCL5 protein is located at 17q11-21 . CCL5 exists in various forms such as monomers, dimers, and multimers. CCL5 is mainly expressed in macrophages, activated T cells, fibroblasts, glial cells, epithelial cells, endothelial cells, platelets and certain tumor cells, and has chemotactic or stimulating effect on various white blood cells such as monocytes, eosinophils, basophils, T lymphocytes, natural killer cells, etc.

CCL3, CCL4, and CCL5 all bind to CCR5 to recruit immune cells, such as immature myeloid dendritic cells, monocytes, macrophages, Th1, Treg, NK, and plasmacytoid dendritic cells, to make them migrate to the inflammatory site or tumor site.

In an embodiment, the engineered immune cell of the present disclosure expresses (i) a chimeric receptor, and (ii) an exogenous CCL3, CCL4 and/or CCL5 gene. More preferably, the engineered immune cell of the present disclosure expresses (i) a chimeric antigen receptor, and (ii) an exogenous CCL4 and/or CCL5 gene.

In an embodiment, the CCL3 used in the present disclosure has at least 70%, preferably at least 80%, more preferably at least 90%, 95%, 97% or 99% or 100% sequence identity to an amino acid sequence represented by SEQ ID NO: 78 or 80, or the coding sequence of CCL3 has at least 70%, preferably at least 80%, more preferably at least 90%, 95%, 97% or 99% or 100% sequence identity to a nucleic acid sequence represented by SEQ ID NO: 77 or 79.

In an embodiment, the CCL4 used in the present disclosure has at least 70%, preferably at least 80%, more preferably at least 90%, 95%, 97% or 99% or 100% sequence identity to an amino acid sequence represented by SEQ ID NO: 82 or 84, or the coding sequence of CCL4 has at least 70%, preferably at least 80%, more preferably at least 90%, 95%, 97% or 99% or 100% sequence identity to a nucleic acid sequence represented by SEQ ID NO: 81 or 83.

In an embodiment, the CCL5 used in the present disclosure has at least 70%, preferably at least 80%, more preferably at least 90%, 95%, 97% or 99% or 100% sequence identity to an amino acid sequence represented by SEQ ID NO: 86 or 88, or the coding sequence of CCL5 has at least 70%, preferably at least 80%, more preferably at least 90%, 95%, 97% or 99% or 100% sequence identity to a nucleic acid sequence represented by SEQ ID NO: 85 or 87.

### Interleukin

Interleukins are a class of cytokines produced by leukocytes and functioning between leukocytes, and play an important role in transmitting information, activating and regulating immune cells, mediating T and B cell activation, proliferation, and differentiation, and inflammatory reactions. Basically, the biological effects of interleukins are achieved by their binding to corresponding receptor, e.g., the biological properties of IL-7 are achieved by the binding of IL-7 to its receptor IL-7R.

In an embodiment, the interleukins that can be used in the present disclosure include, but are not limited to, IL-2, IL-7, IL-12, IL-15, IL-21, IL-17, IL-18, IL-23, IL-33, or a subunit thereof, or a combination thereof, or a combination of subunits thereof, more preferably IL-7 or a subunit thereof. IL-2 is mainly produced by T cells and function by autocrine and paracrine. IL-2 not only can maintain T cell growth, promote the production of cytokines, but also can induce CD8+ T cells and CD4+ T cells to exert cytotoxic effect. In addition, IL-2 also can stimulate NK cell to proliferate, enhance the NK killing activity, promote the NK cells to produce factors such as IFNγ, TNFβ, and TGFβ. IL-2 can also activate macrophages, and enhance the antigen presenting ability and the target cell killing ability of the macrophages. IL-7 is mainly produced by bone marrow and thymic stromal cells, and its main functions relate to the following aspects: promoting precursor B cell growth; inhibiting peripheral T cell apoptosis, inducing cell proliferation, and sustained survival; and affecting the development and function of dendritic cells and macrophages, and inducing macrophages to secrete multiple cytokines. IL-12 mainly acts on T cells and NK cells. Specifically, IL-12 can stimulate proliferation of activated T cells, and promote differentiation of Th0 cells to Th1 cells; induce cytotoxic activities of CTL and NK cells and promote the same to secrete cytokines such as IFNγ, TNFα, and GMCSF; and promote expression of NK cells and IL-2Rα, TNF receptors, and CD56, and enhance an ADCC effect on tumor cells. IL-15 can be produced by a variety of cells, such as activated macrophages, epidermal cells, and fibroblasts. As the molecular structure of IL-15 is similar to that of IL-2, it can bind to target cells by using the β chain and γ chain of IL-2R, and exert a biological activity similar to that of IL-2, for example, stimulating proliferation of T cells and NK cells, and inducing proliferation and differentiation of B cells. IL-21 is produced by activated CD4+ T cells, NKT cells, Tfh cells, and Th17 cells, and has high homology with IL-2 and IL-15. IL-21 has a wide range of immunomodulatory functions. Its activation can enhance proliferation of activated CD8+ T cells, enhance the cytotoxic activity of NK cells, and promote proliferation and differentiation of B cells. IL-17 is secreted by Th17 cells, and is a proinflammatory molecule. It can inhibit regulatory T cells, and recruit and activate neutrophils and macrophages. IL-18 is also a proinflammatory molecule and is secreted by T cells and NK cells. Activated macrophages also secrete a large amount of IL-18. It has been reported that IL-18 plays an important role in innate immunity and adoptive immunity. IL-23 is likewise secreted by T cells and NK cells, and IL-23 can promote Th17 cell differentiation. Dendritic cells, monocytes, and macrophages also express IL-23 receptors at a low-level, and can be activated by IL-23. IL-33 has a sequence similar to that of IL-18. IL-33 binds to the receptor ST2 through the IL-1 receptor auxiliary protein IL-RAcP, forming a heterodimer that activates NF-κB, MAPK and other signaling pathways, and strongly induces the production of pro-inflammatory factors and chemokines to exert biological effects. IL-33 is expressed in a variety of cells, including epithelial cells, fibroblasts, macrophages, dendritic cells, etc. Studies have shown that IL-33 can stimulate immature dendritic cells to produce Treg, promote Th1 cells to secrete IFN-γ, increase the expression of CD69, thereby activating NK, NKT cells and CD8+ T cells, and promoting anti-tumor immunity. Studies have shown that these interleukins alone or in combination (such as IL-33 and IL-12, IL-7+IL-12, IL-7+IL15, etc.) can exert effective anti-tumor effects.

In an embodiment, the interleukin used in the present disclosure has at least 70%, preferably at least 80%, more preferably at least 90%, 95%, 97%, or 99% or 100% sequence identity to an amino acid sequence represented by SEQ ID NO: 42, 44, 46, 48, 50, 52, 54, 56, 58, 60, 62, 64, 66, 68, 70, 72, 74, 76, or an encoding sequence thereof has at least 70%, preferably at least 80%, more preferably at least 90%, 95%, 97%, or 99% or 100% sequence identity to a nucleic acid sequence represented by 41 , 43, 45, 47, 49, 51, 53, 55, 57, 59, 61, 63, 65, 67, 69, 71, 73, 75.

### Expression of exogenous gene

Expression of the exogenous gene in the present disclosure, e.g., interleukin, CCL3, CCL4, CCL5, can be constitutive expression or conditional expression.

In an embodiment, the expression of the exogenous interleukin, CCL3, CCL4, CCL5 is conditional expression. For example, the exogenous gene of the present disclosure may be operably linked to an inducible, repressible, or tissue-specific promoter, as required, so as to regulate the expression level of the introduced exogenous gene at particular time or in a particular tissue, and cell type. In an embodiment, the promoter is an inducible promoter, i.e., a promoter that initiates transcription only in the presence of specific environmental conditions, developmental conditions, or inducers. Such environmental conditions include, for example, tumor acidic microenvironments, tumor hypoxic microenvironments, etc. Such inducers include, for example, doxycycline, tetracycline, or analogues thereof, wherein the analogues of tetracycline include, for example, chlortetracycline, oxytetracycline, demethylchlortetracycline, methacycline, doxycycline, and minocycline. Inducible promoters include, for example, Lac operon sequence, tetracycline operon sequence, galactose operon sequence, or doxycycline operon sequence. In another embodiment, the promoter is a repressible promoter, i.e., the expression of the exogenous gene in the cell is inhibited or the exogenous gene is not expressed in the presence of a repressor specific for the repressible promoter. Repressible promoter includes, for example, Lac repressible elements or tetracycline repressible elements. Inducible/repressible expression systems well known to those skilled in the art can be used in the present disclosure, including, but not limited to, Tet-on system, Tet-off system, Cre/loxP system, etc.

In an embodiment, the interleukin, CCL3, CCL4, CCL5 may be operably linked to a localization domain, wherein the localization domain can locate the exogenous gene of the present disclosure at a specific cellular position for expression, for example, a cell membrane, a specific organelle in the cytoplasm, e.g., endoplasmic reticulum, golgi apparatus, nucleus, etc. The localization domain includes, but is not limited to, a nuclear localization signal, a leader peptide, a transmembrane domain, and the like. In an embodiment, the exogenous genes, i.e., interleukin, CCL3, CCL4, CCL5 of the present disclosure are operably linked to the transmembrane domain, so as to be anchored on the surface of the engineered immune cell to be expressed.

In an embodiment, the exogenous gene of the present disclosure, e.g., interleukin, CCL3, CCL4, CCL5 protein, may be wildtype or a fusion protein or a mutant with specific properties (e.g., resistant to protease hydrolysis).

### Nucleic acid

The present disclosure further provides a nucleic acid molecule, which contains (i) a nucleic acid sequence encoding a chimeric receptor, and (ii) a nucleic acid sequence encoding CCL3, CCL4 and/or CCL5.

In an embodiment, the nucleic acid molecule further comprises (iii) a nucleic acid sequence encoding an interleukin. Preferably, the interleukins include but are not limited to IL-2, IL-7, IL-12, IL-15, IL-21, IL-17, IL-18, IL-23, IL33, a subunit thereof, or a combination thereof, or a combination of subunits thereof, more preferably IL-7 or a subunit thereof.

In an embodiment, the chimeric receptor is a T cell receptor or a chimeric antigen receptor, preferably a chimeric antigen receptor. Definition of the chimeric antigen receptor is as described in the above.

As used herein, the term "nucleic acid molecule" includes a sequence of ribonucleotide and deoxyribonucleotide, such as modified or unmodified RNA or DNA, each in single-stranded and/or double-stranded form, linear or circular, or their mixtures (including hybrid molecules). Thus, the nucleic acid according to the present disclosure includes DNA (e.g. dsDNA, ssDNA, cDNA), RNA (e.g. dsRNA, ssRNA, mRNA, ivtRNA), their combinations or derivatives (e.g. PNA). Preferably, the nucleic acid is DNA or RNA, more preferably mRNA.

The nucleic acid may contain a conventional phosphodiester bond or an unconventional bond (e.g., amide bond, such as found in peptide nucleic acid (PNA)). The nucleic acid of the present disclosure further may contain one or more modified bases, such as, for example, trityl base and uncommon base (such as inosine). Other modifications also can be contemplated, including chemical, enzymatic, or metabolic modifications, so long as the multi-chain CAR of the present disclosure can be expressed from polynucleotides. The nucleic acid can be provided in isolated form. In an embodiment, the nucleic acid also may include a regulatory sequence, such as a transcriptional control element (including a promoter, an enhancer, an operon, a repressor, and a transcription termination signal), ribosome binding sites, and introns.

The nucleic acid sequences of the present disclosure can be codon-optimized for optimal expression in a desired host cell (e.g., immune cell); or for expression in a bacterial, yeast, or insect cell. Codon optimization refers to substitution of a codon in the target sequence that is generally rare in highly expressed genes of a given species with a codon that is generally common in highly expressed genes of such species, and the codons before and after the substitution encode the same amino acid. Therefore, the selection of an optimal codon depends on the codon usage preference of the host genome.

### Vector

The present disclosure further provides a vector, containing the nucleic acid of the present disclosure. In the above, a nucleic acid sequence encoding a chimeric receptor, optionally a nucleic acid sequence encoding IL-7, a nucleic acid sequence encoding CCL3, a nucleic acid encoding CCL4, and/or a nucleic acid encoding CCL5 can be located in one or more vectors.

As used herein, the term "vector" is an intermediary nucleic acid molecule used to transfer (exogenous) genetic material into a host cell, and in the host cell the nucleic acid molecule can be, for example, replicated and/or expressed.

The vector generally includes targeting vectors and expression vectors. The "targeting vector" is a medium that delivers an isolated nucleic acid to the interior of a cell by, for example, homologous recombination or by using a hybrid recombinase of a sequence at specific target site. The "expression vector" is a vector used for transcription of heterologous nucleic acid sequences (for example, those sequences encoding the chimeric antigen receptor polypeptides of the present disclosure) in suitable host cells and the translation of their mRNAs. Suitable vectors that can be used in the present disclosure are known in the art, and many are commercially available. In an embodiment, the vector of the present disclosure includes, but is not limited to, plasmid, virus (e.g., retrovirus, lentivirus, adenovirus, vaccinia virus, Rous sarcoma virus (RSV), polyoma virus, and adeno-associated virus (AAV), etc.), bacteriophage, phagemid, cosmid, and artificial chromosome (including BAC and YAC). The vector itself is usually a nucleotide sequence, and usually is a DNA sequence containing an insert (transgene) and a larger sequence as "backbone" of the vector. Engineered vector typically also contains an origin autonomously replicating in the host cell (if stable expression of polynucleotide is desired), a selectable marker, and a restriction enzyme cleavage site (e.g., a multiple cloning site, MCS). The vectors may additionally contain elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an *in vitro* transcription vector.

### Engineered immune cell and preparation method thereof

The present disclosure further provides an engineered immune cell, which contains the nucleic acid or the vector of the present disclosure. In other words, the engineered immune cells of the present disclosure express a chimeric receptor (e.g., a chimeric antigen receptor), CCL3, CCL4 and/or CCL5 gene, and optionally an interleukin, such as IL-7.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, an NK cell, and/or an NKT cell, or an immune cell derived from a stem cell, such as an adult stem cell, an embryonic stem cell, a cord blood stem cell, a progenitor cell, a bone marrow stem cell, an induced pluripotent stem cell, a totipotent stem cell or a hematopoietic stem cell. Preferably, the immune cell is a T cell. The T cell may be any T cell, such as *in vitro* cultured T cell, for example, primary T cell, or T cell from *in vitro* cultured T cell line, e.g., Jurkat, SupT1, etc., or T cell obtained from a subject. Examples of a subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be at any stage of development including, but not limited to, a CD4+/CD8+ T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell, etc. In a preferred embodiment, the immune cell is a human T cell. The T cell can be obtained from the blood of a subject using a variety of techniques known to those of skill in the art, such as Ficoll isolation. In the present disclosure, the immune cell is engineered to express the chimeric antigen receptor and the exogenous CCL3, CCL4 and/or CCL5 gene, and optionally an interleukin, such as IL-7.

The nucleic acid sequence encoding a chimeric receptor (e.g. chimeric antigen receptor) polypeptide and a CCL3, CCL4 and/or CCL5 gene, and an optional interleukin gene, such as IL-7, can be introduced into an immune cell using conventional methods known in the art (e.g., by transduction, transfection, transformation). "Transfection" is a process of introducing a nucleic acid molecule or polynucleotide (including a vector) into a target cell. An example is RNA transfection, i.e., the process of introducing RNA (such as *in vitro* transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation.

Therefore, the present disclosure further provides a method of preparing an engineered immune cell, including introducing the following into the immune cell: (a) a first nucleic acid sequence encoding a chimeric receptor or the chimeric receptor encoded thereby; b) a second nucleic acid sequence encoding CCL3, CCL4 and/or CCL5 or a CCL3, CCL4 and/or CCL5 protein encoded thereby; and optionally (c) a third nucleic acid sequence encoding an interleukin or the interleukin encoded thereby.

In an embodiment, the above components (a), (b), and (c) can be introduced in sequence into the immune cell in any order. In another embodiment, the above components (a), (b), and (c) can be simultaneously introduced into the immune cell, e.g., cloning (a), (b), and (c) in one or more vectors.

After introducing the nucleic acid or vector into the immune cell, a person skilled in the art could amplify and activate the resulting immune cell by conventional techniques.

In an embodiment, the engineered immune cells of the present disclosure may further comprise suppressed or silenced expression of at least one gene selected from the group consisting of CD52, GR, dCK, TCR/CD3 gene (e.g. TRAC, TRBC, CD3γ, CD3δ, CD3ε, CD3ζ), MHC related gene (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and an immune checkpoint gene such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1 B2, and GUCY1 B3.

Methods of inhibiting gene expression or silencing genes are well known to those skilled in the art. For example, antisense RNA, RNA decoy, RNA aptamer, siRNA, shRNA/miRNA, trans dominant negative protein (TNP), chimeric/fusion protein, chemokine ligand, anti-infective cellular protein, intrabody (sFv), nucleoside analog (NRTI), non-nucleoside analog (NNRTI), integrase inhibitor (oligonucleotide, dinucleotide, and chemical agent) and protease inhibitor may be used to inhibit gene expression. Further, DNA breakage mediated by a meganuclease, a zinc finger nuclease, a TALE nuclease, or a Cas enzyme in a CRISPR system may also be used for silencing a gene.

### Kit and pharmaceutical composition

The present disclosure provides a kit, which contains the engineered immune cell, the nucleic acid molecule, or the vector of the present disclosure.

In a preferred embodiment, the kit of the present disclosure further contains instructions.

The present disclosure further provides a pharmaceutical composition, which contains the engineered immune cell, the nucleic acid molecule, or the vector of the present disclosure as an active agent, and one or more pharmaceutically acceptable excipients. Therefore, the present disclosure further encompasses use of the nucleic acid molecule, the vector or the engineered immune cell in the preparation of a pharmaceutical composition or medicine.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, cosolvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the application is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition containing, for example, the immune cell as described herein is generally provided in a form of solution, and preferably contains a pharmaceutically acceptable buffer agent.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210 and 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic use

The present disclosure further provides a method of treating a subject with cancer, infection or autoimmune disease, including administering to the subject an effective amount of the immune cell or the pharmaceutical composition according to the present disclosure. Therefore, the present disclosure also encompasses use of the nucleic acid molecule, vector, engineered immune cell and pharmaceutical composition in the preparation of a medicine for treating cancer, infection, or autoimmune diseases.

In an embodiment, an effective amount of the immune cell and/or the pharmaceutical composition of the present disclosure is directly administered to the subject.

In another embodiment, the treatment method of the present disclosure is *ex vivo* treatment. Specifically, the method includes the steps of: (a) providing a sample, the sample containing an immune cell; (b) introducing the chimeric receptor (such as chimeric antigen receptor) of the present disclosure and an exogenous gene to be expressed into the immune cell *in vitro* to obtain a modified immune cell, and (c) administering the modified immune cell to the subject in need thereof. Preferably, the immune cell provided in step (a) is selected from a macrophage, a dendritic cell, a monocyte, a T cell, an NK cell, and/or an NKT cell; and the immune cell can be obtained from the sample (particularly a blood sample) of the subject by conventional methods known in the art. However, other immune cells capable of expressing the chimeric antigen receptor and exogenous gene of the present disclosure and exerting the desired biological effect function as described herein also can be used. Besides, the immune cells generally selected are compatible with the subject's immune system, i.e., it is preferred that the immune cells do not trigger an immunogenic response. For example, a "universal recipient cell", i.e., a universally compatible lymphocyte exerting a desired biological effect function and being capable of growing and amplifying *in vitro,* can be used. The use of such cells will not require obtaining and/or providing the subject's own lymphocyte. The *ex vivo* introduction of step (c) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods include using a transfection reagent (such as a liposome) or transient RNA transfection.

In an embodiment, the immune cell is an autologous or allogeneic cell, preferably T cell, macrophage, dendritic cell, monocyte, NK cell and/or NKT cell, more preferably T cell, NK cell or NKT cell.

As used herein, the term "autologous" means that any material derived from an individual will be later re-introduced into the same individual.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic material from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse, or a cow. Mammals other than human can be advantageously used as subjects representing cancer animal models. Preferably, the subject is a human.

In an embodiment, the cancer is a cancer associated with expression of the target to which the ligand binding domain binds. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (such as small cell lung cancer, non-small cell lung cancer, lung adenocarcinoma and squamous lung cancer), lymphoma (including Hodgkin's lymphoma and non-Hodgkin's lymphoma), melanoma, myeloma, neuroblastoma, oral cancer (e.g., lips, tongue, mouth, and pharynx), ovarian cancer, pancreatic cancer, prostate cancer, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer and other cancers and sarcomas, and B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, and Waldenstrom macroglobulinemia, chronic lymphocytic leukemia (CLL), acute lymphocytic leukemia (ALL), B cell acute lymphocytic leukemia (B-ALL), T cell acute lymphocytic leukemia (T-ALL), B cell prolymphocytic leukemia, blast cell plasmacytoid dendritic cell tumor, Burkitt lymphoma, diffuse large B cell lymphoma, follicular lymphoma, chronic myelogenous leukemia (CML), malignant lymphoproliferative disorder, MALT lymphoma, hairy cell leukemia, marginal zone lymphoma, multiple myeloma, myelodysplasia, plasmablastic lymphoma, preleukemia, plasmacytoid dendritic cell tumor, post-transplant lymphoproliferative disorder (PTLD), and other diseases associated with target expression. Preferably, the disease which can be treated with the engineered immune cell or the pharmaceutical composition of the present disclosure is selected from the group consisting of: leukemia, lymphoma, multiple myeloma, brain glioma, pancreatic cancer, gastric cancer, and so on.

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

In an embodiment, the method further includes administering to the subject one or more additional chemotherapeutic agents, biological agents, medicines, or treatments. In this embodiment, the chemotherapeutic agents, biological agents, medicines, or treatments are selected from the group consisting of radiotherapy, surgery, antibody reagent and/or small molecule and any combination thereof.

The present disclosure will be described in detail below with reference to the accompanying drawings and examples. It should be noted that those skilled in the art should understand that the accompanying drawings of the present disclosure and examples thereof are only for illustrative purpose, and cannot constitute any limitation to the present disclosure. The examples of the present disclosure and the features in the examples may be combined with each other without contradiction.

### Specific Embodiments

### Example 1 Construction of mouse pancreatic cancer cell line Panc02-mCD19

### 1. Preparation of pLV-mCD19 plasmid

With mouse spleen total mRNA as a template, a mouse spleen total cDNA sequence was obtained by reverse transcription PCR, and then a mouse mCD19 sequence containing Xbal and Sall restriction sites was obtained by PCR. The mCD19 gene was then recombined into a pLVX vector (PPL, Cat No.: PPL00157-4a), to obtain a pLV-mCD19 plasmid.

### 2. Lentivirus packaging

In a T175 culture flask, 293T cells were inoculated in 30 ml of DMEM medium containing 10% fetal bovine serum at a density of 30×10⁶ cells/flask, and incubated overnight in an incubator at 37 °C and 5% CO₂.

In a sterile tube, 3 ml of Opti-MEM (Gibco, Lot No. 31985-070), 34 µg of pLV-BHAm-mCD19 plasmid, 8.5 µg of pMD2. G vector (Addgene, Lot No. 12259), and 17 µg of psPAX2 vector (Addgene, Lot No. 12260) were added. Then, 120 µl of X-treme GENE HP DNA transfection reagent (Roche, Lot. No. 06366236001) was added, well mixed immediately, followed by incubation at room temperature for 15 min. Then the plasmid/vector/transfection reagent mixture was added dropwise into the culture flask of 293T cells prepared in advance, and cultured overnight under a condition of 5% CO₂ at 37°C. Cultures were collected 24 hours and 48 hours after transfection, and after combination, ultracentrifugation (25000 g, 4 °C, 2.5 h) was performed to obtain a concentrated pLV-BHAm-mCD19 lentivirus, which was stored at -80 °C.

### 3. Screening of Panc02-mCD19 cell line

RPMI-1640 medium (Gibco, Lot No. C12430500BT) containing 10% fetal bovine serum, 1×10⁶ mouse pancreatic cancer cells Panc02 (donated by laboratory of China Pharmaceutical University), and 200 µl of pLV-BHAm-mCD19 lentivirus were added to each well of a 6-well cell culture plate, and incubated at 37°C under a condition of 5% CO₂ for 48 h. Cells were then digested with 0.25% pancreatin into single cell suspension, and diluted and transferred to a 96-well plate for continued culture, until monoclonal cells appeared. Monoclonal cells were picked, digested again with 0.25% pancreatin into single cells, and the cells were resuspended with 200 µl of opti-MEM medium. Infection efficiency was detected by flow cytometry with APC antimouse CD19 antibody (Biolegend, Lot No. 115512), and CD19 positive clones were screened. After the positive clones were passaged 3-4 times, CD19 expression level was detected by flow cytometry. Panc02 cells not infected by virus were used as control.

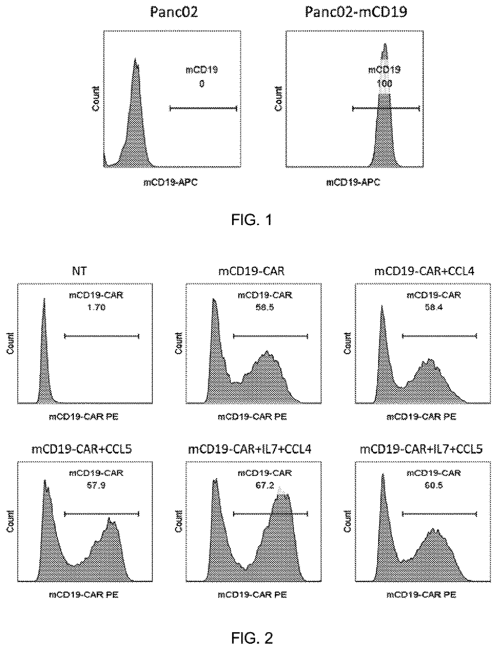

Results are as shown in FIG. 1. In the finally screened Panc02-mCD19 cell line, the CD19 expression rate is 100%.

### Example 2. Preparation of CAR-T cell

### 1. Construction of retroviral plasmid

Encoding sequence fragments of CD19-scFv (SEQ ID NO: 26), CD8a hinge region and transmembrane region (SEQ ID NO: 35 and 29), 41bb intracellular domain (SEQ ID NO: 31) and CD3 ζ intracellular domain (SEQ ID NO: 33) successively linked were artificially synthesized, and an Xhol/EcoRI enzyme sites were added at two ends. The fragment was cloned into an MSCV vector, to obtain an MSCV-mCD1 9-CAR plasmid.

Encoding sequence fragments of T2A (SEQ ID NO: 37) and IL-7 (SEQ ID NO: 43) successively linked were artificially synthesized, and an EcoRI/Sall enzyme sites were added at two ends. The fragment was cloned into the MSCV-mCD1 9-CAR vector, to obtain an MSCV-mCD19-CAR-IL-7 plasmid.

Encoding sequence fragments of T2A (SEQ ID NO: 37) and CCL4 (SEQ ID NO: 83) successively linked were artificially synthesized, and an EcoRI/Sall enzyme sites were added at two ends. The fragment was cloned into the MSCV-mCD1 9-CAR vector, to obtain an MSCV-mCD1 9-CAR-CCL4 plasmid.

Encoding sequence fragments of T2A (SEQ ID NO: 37) and CCL5 (SEQ ID NO: 87) successively linked were artificially synthesized, and an EcoRI/Sall enzyme site was added at two ends. The fragment was cloned into the MSCV-mCD19-CAR vector, to obtain an MSCV-mCD1 9-CAR-CCL5 plasmid.

### 2. Preparation of retrovirus

In a T175 culture flask, 293T cells were inoculated in 30 ml of DMEM medium containing 10% fetal bovine serum at a density of 30×10⁶ cells/flask, and incubated overnight in an incubator at 37 °C and 5% CO₂ for viral packaging.

In a sterile tube, 3 ml of Opti-MEM (Gibco, Lot No. 31985-070), 45 µg of retrovirus plasmid (MSCV-mCD19-CAR plasmid, MSCV-mCD19-CAR-IL-7 plasmid, MSCV-mCD19-CAR-CCL4 plasmid, or MSCV-mCD19-CAR-CCL5 plasmid), and 15 µg of packaging vector pCL-Eco (Shanghai Hebio Biotechnology Co., Ltd, Lot No. P3029) were added. Then, 120 µl of X-treme GENE HP DNA transfection reagent (Roche, Lot. No. 06366236001) was added, well mixed immediately, followed by incubation at room temperature for 15 min. Then the plasmid/vector/transfection reagent mixture was added dropwise into the culture flask of 293T cells prepared in advance, and cultured overnight under a condition of 5% CO₂ at 37 °C. Cultures were collected 72 hours after transfection, and centrifuged (2000 g, 4 °C, 10 min) to obtain retrovirus supernatant.

### 3. Preparation of CAR-T cell

T lymphocytes were isolated from mouse spleens, and the T cells were activated with DynaBeads CD3/CD28 CTS^{™} (Gibco, Lot. No. 40203D), and cultured in 5% CO₂ at 37 °C for 1 day.

The activated T cells were inoculated into a 24-well plate pre-coated with RetroNectin overnight at a density of 3×10⁶ cells/mL per well, then 500 µL of complete medium (NT, control), MSCV-mCD19-CAR virus, MSCV-mCD19-CAR-CCL4 virus, MSCV-mCD19-CAR-CCL5 virus, MSCV-mCD19-CAR-IL-7 virus + MSCV-mCD19-CAR-CCL4 virus, or MSCV-mCD19-CAR-IL-7 virus + MSCV-mCD19-CAR-CCL5 virus was added, respectively, and the complete medium was supplemented to 2 mL.

The 24-well plate was placed in a centrifuge for spin infection, and centrifuged at 2000 g at 32 °C for 2 h. Then, the 24-well plate was immediately placed in a CO₂ incubator at 37 °C for static culture. The medium was replaced with fresh medium the next day, and the cell density was adjusted to 1×10⁶ cells/mL. Three days after infection, the cells were collected for subsequent analysis. The collected cells were NT cells, mCD19-CAR cells, mCD19-CAR+CCL4 cells, mCD19-CAR+CCL5 cells, mCD19-CAR+IL7+CCL4 cells, and mCD19-CAR+IL7+CCL5 cells.

### Example 3. Detection of expression of CAR-T cell

### 1. Expression level of CAR on cell surface

2×10⁵ CAR-T cells prepared in Example 2 were sampled, and the expression level of CAR on CAR T cells was detected by flow cytometry with Goat Anti-Rat IgG (H&L) Biotin (BioVision, Lot No. 6910-250) as a primary antibody, and APC Streptavidin (BD Pharmingen, Lot No. 554067) as a secondary antibody. Results are as shown in FIG. 2.

It can be seen that compared with the control, the CAR positive efficiency of mCD19-CAR cells, mCD19-CAR+CCL4 cells, mCD19-CAR+CCL5 cells, mCD19-CAR+IL7+CCL4 cells, and mCD19-CAR+IL7+CCL5 cells is about 60%, indicating that all these cells can effectively express CAR.

### 2. Expression level of IL-7

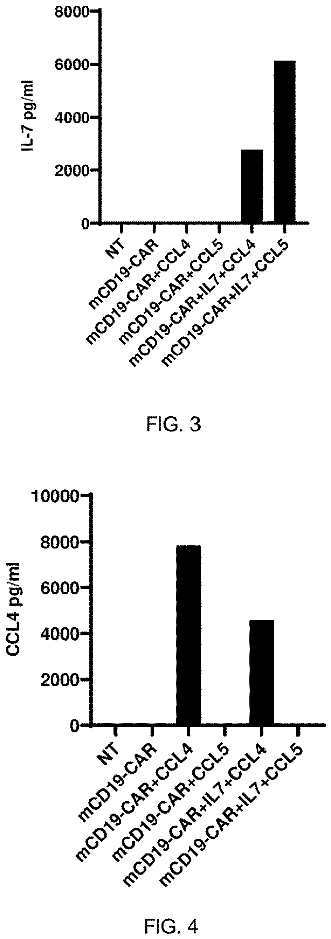

The supernatant of the CAR-T cells prepared in Example 2 was collected, and the IL-7 secretion level in the cells was detected with a Mouse IL-7 DuoSet ELISA kit (R&D Systems, Lot No. DY407) according to the manufacturer's recommendations. Results are as shown in FIG. 3.

It can be seen that two CAR T cells transfected with MSCV-mCD19-CAR-IL-7 virus can efficiently express IL-7.

### 3. Expression level of CCL4

The supernatant of the CAR-T cells prepared in Example 2 was collected, and the CCL4 secretion level in the cells was detected with a Mouse CCL4 DuoSet ELISA kit (R&D Systems, Lot No. DY451) according to the manufacturer's recommendations. Results are as shown in FIG. 4.

It can be seen that two CAR T cells transfected with MSCV-mCD19-CAR-CCL4 virus can efficiently express CCL4.

### 4. Expression level of CCL5

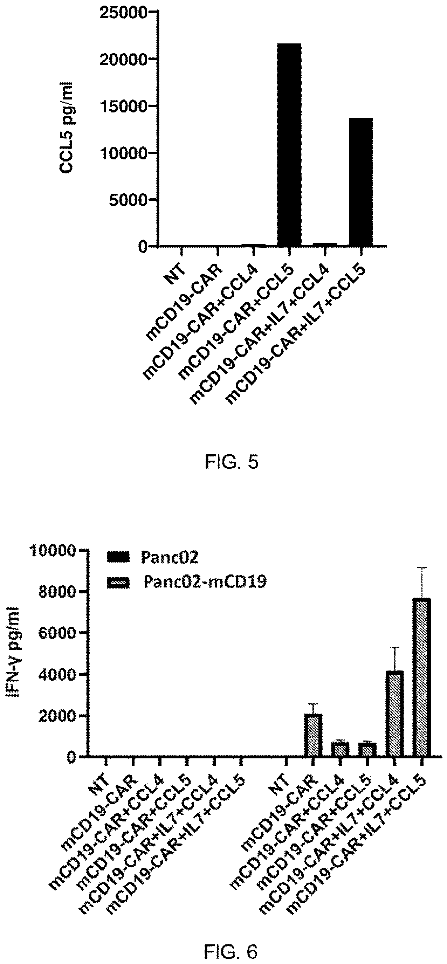

The supernatant of the CAR-T cells prepared in Example 2 was collected, and the CCL5 secretion level in the cells was detected with a Mouse CCL5 DuoSet ELISA kit (R&D Systems, Lot No. DY478) according to the manufacturer's recommendations. Results are as shown in FIG. 5.

It can be seen that two CAR T cells transfected with MSCV-mCD19-CAR-CCL5 virus can efficiently express CCL5.

### Example 4. Detection of IFN-γ secretion level of CAR-T cell

The NT cells, mCD19-CAR cells, mCD19-CAR+IL-7 cells, mCD19-CAR+CCL4 cells, mCD19-CAR+CCL5 cells, mCD19-CAR+IL7+CCL4 cells, and mCD19-CAR+IL7+CCL5 cells were added to a 96-well round bottom plate at a concentration of 2×10⁵ cells/100 µl, respectively. Target Panc02-mCD19 cells or non-target Panc02 cells were then added to each well at a concentration of 1×10⁴ cells/100 µl, respectively. Culture supernatant was collected after 24 h of culture at 37 °C. The expression level of IFN-γ in culture supernatant was detected with a Mouse IFN-gamma DuoSet ELISA kit (R&D, Lot No. DY485) according to the manufacturer's recommendations.

The detection result is as shown in FIG. 6. It can be seen that no release of IFN-γ is detected in the non-target cell Panc02, and the NT cell does not express IFN-γ, indicating that killing of the CAR T cell in the present example is specific. Moreover, when killing target cells, compared with T cell expressing only CAR, additional expression of CCL4 or CCL5 alone decreased IFN-γ release levels, whereas expression of IL-7+CCL4 or IL-7+CCL5 increased IFN-γ release levels.

### Example 5. Verification of tumor inhibition effect of CAR-T cell

5×10⁵ Panc02-mCD19 pancreatic cancer cells prepared in Example 1 were subcutaneously inoculated in left-forelimb axilla region of healthy C57BL/6 mice.

The mice inoculated with pancreatic cancer cells were randomly divided into 6 groups, with 5 mice in each group. When the tumor volume reaches 100 mm³, each mouse was injected with 1×10⁶ NT cells, mCD19-CAR cells, mCD19-CAR+CCL4 cells, mCD19-CAR+CCL5 cells, mCD19-CAR+IL7+CCL4 cells, or mCD19-CAR+IL7+CCL5 cells prepared in Example 2 through tail vein.

Changes of body weight and tumor volume of the mice were monitored until the end of the experiment.

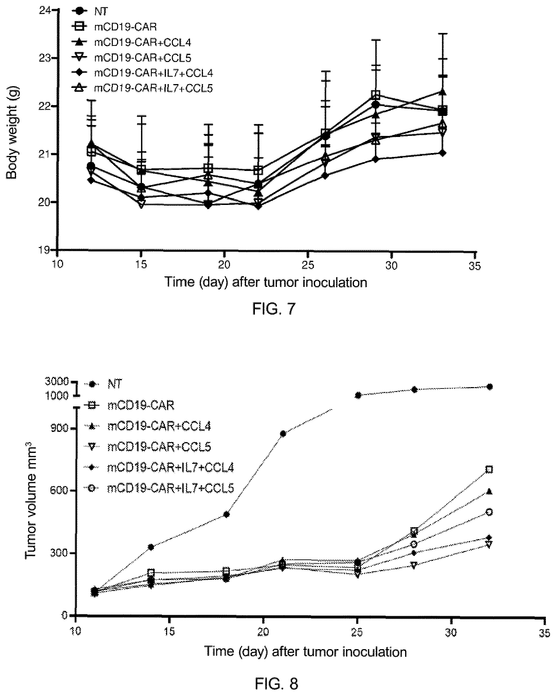

The changes of body weight of the mice are shown in FIG. 7. It can be seen that after the administration of CAR-T cells, the body weight of each group of mice is not significantly different compared with that of the control group, and in the observation period, when the tumor of the mice does not exceed 1500 mm³, the mice are active and have a normal hair color, which indicates that the administration of CAR-T cells will not have obvious toxic side effects on the mice.

The changes of tumor volume of the mice are shown in FIG. 8. It can be seen that compared with the NT cells and the conventional CAR-T cells, the CAR-T cells expressing only CCL4 or CCL5 can enhance the anti-tumor effect, indicating that the CCL4 or CCL5 alone can exhibit a synergistic effect with the CAR-T cells. Besides, the inventors also unexpectedly found that further expression of IL-7 can significantly increase the promoting effect of CCL4 on CAR-T cells, thereby more significantly inhibiting tumor growth. In contrast, the promoting effect of IL-7 on CCL5 was lower than that on CCL4, but the combination of IL-7+CCL5+CAR was still slightly better than CAR alone in inhibiting the tumor.

The above results show that co-expression of CCL4, CCL5 or their combination with IL-7 can effectively enhance the inhibitory effect of CAR-expressing engineered immune cells on target pancreatic cancer cells.

### Example 6. Verification of the tumor inhibition effect of CAR-T cells expressing CCL3

According to the method described in Example 2, CAR-T cells expressing CCL3 were prepared, wherein the coding sequence fragments of T2A (SEQ ID NO: 37) and CCL3 (SEQ ID NO: 79) were cloned into the MSCV-mCD1 9-CAR vector to obtain MSCV-mCD19-CAR-CCL3 plasmid and packaged to form retrovirus. Activated T cells were transfected with the virus to obtain mCD19-CAR+CCL3 cells. Activated T cells were transfected with both MSCV-mCD1 9-CAR-CCL3 virus and MSCV-mCD19-CAR-IL-7 virus to obtain mCD19-CAR+IL7+CCL3 cells.

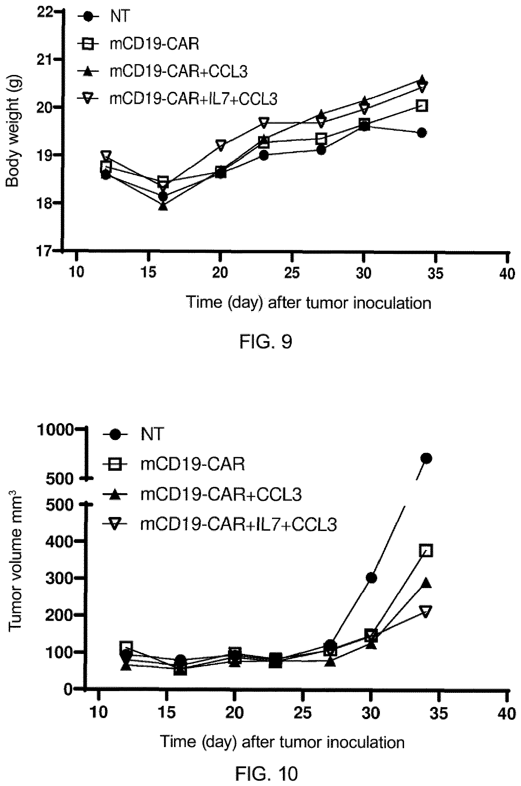

According to the method described in Example 5, the *in vivo* inhibitory effect of CAR-T cells on tumors was verified. Figure 9 shows the body weight changes of the mice, indicating that the administration of CAR-T cells will not have obvious toxic side effects on the mice. Figure 10 shows the tumor volume changes in mice. It can be seen that CCL3 alone has a synergistic effect with CAR-T cells, and further expression of IL7 significantly increases the promoting effect of CCL3 on CAR-T cells, thereby inhibiting tumor growth more significantly.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. An engineered immune cell, expressing (i) a chimeric receptor, and (ii) an exogenous CCL3, CCL4 and/or CCL5 gene.

2. The engineered immune cell according to claim 1, **characterized in that** the CCL3 gene has at least 90% identity to a nucleic acid sequence represented by SEQ ID NO: 77 or 79, or a polypeptide encoded by the CCL3 gene has at least 90% identity to an amino acid sequence represented by SEQ ID NO: 78 or 80; the CCL4 gene has at least 90% identity to a nucleic acid sequence represented by SEQ ID NO: 81 or 83, or a polypeptide encoded by the CCL4 gene has at least 90% identity to an amino acid sequence represented by SEQ ID NO: 82 or 84; the CCL5 gene has at least 90% identity to a nucleic acid sequence represented by SEQ ID NO: 85 or 87, or a polypeptide encoded by the CCL5 gene has at least 90% identity to an amino acid sequence represented by SEQ ID NO: 86 or 88.

3. The engineered immune cell according to any one of claims 1-2, **characterized in that** the engineered immune cell further expresses (iii) an exogenous interleukin.

4. The engineered immune cell according to claim 3, **characterized in that** the interleukin is IL-2, IL-7, IL-12, IL-15, IL-21, IL-17, IL-18, IL-23, IL-33, or a subunit thereof, or a combination thereof, or a combination of subunits thereof.

5. The engineered immune cell according to claim 4, **characterized in that** an encoding gene of the interleukin has at least 90% identity to a nucleic acid sequence represented by SEQ ID NO: 41, 43, 45, 47, 49, 51, 53, 55, 57, 59, 61, 63, 65, 67, 69, 71, 73, or 75, or the interleukin has at least 90% identity to an amino acid sequence represented by SEQ ID NO: 42, 44, 46, 48, 50, 52, 54, 56, 58, 60, 62, 64, 66, 68, 70, 72, 74, or 76.

6. The engineered immune cell according to any one of claims 1-5, **characterized in that** the chimeric receptor is a chimeric antigen receptor or a T cell receptor.

7. The engineered immune cell according to claim 6, **characterized in that** the chimeric receptor is a chimeric antigen receptor comprising: a ligand binding domain, a transmembrane domain, a co-stimulatory domain, and an intracellular signaling domain.

8. The engineered immune cell according to claim 7, **characterized in that** the ligand binding domain is selected from the group consisting of an immunoglobulin molecule, Fab, Fab', F(ab')2, Fv fragment, scFv, disulfide bond-linked Fv (sdFv), heavy chain variable region (VH) or light chain variable region (VL) of an antibody, Fd fragment consisting of VH and CH1 domains, linear antibody, single domain antibody, nanobody, and non-immunoglobulin antigen binding scaffold.

9. The engineered immune cell according to claim 7 or 8, **characterized in that** the ligand binding domain binds to a target selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof.

10. The engineered immune cell according to any one of claims 7-9, **characterized in that** the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137 and CD154.

11. The engineered immune cell according to any one of claims 7-10, **characterized in that** the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d.

12. The engineered immune cell according to any one of claims 7-11, **characterized in that** the co-stimulatory domain is one or more co-stimulatory signaling domains of a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, CD94, LTB, ZAP70 and a combination thereof.

13. The engineered immune cell according to any one of claims 1-12, **characterized in that** the immune cell further contains at least one inactive gene selected from the group consisting of CD52, GR, TCR α, TCR β, CD3 γ, CD3 δ, CD3 ε, CD247 ζ, HLA-I, HLA-II, B2M, PD1, CTLA-4, LAG3 and TIM3.

14. The engineered immune cell according to any one of the claims 1-13, **characterized in that** expression or the CCL3, CCL4, ana/or CCL5 gene is a conditional expression.

15. The engineered immune cell according to any one of claims 1-13, **characterized in that** the CCL3, CCL4, and/or CCL5 gene is operably linked to a localization domain.

16. The engineered immune cell according to any one of claims 1-15, **characterized in that** the immune cell is selected from the group consisting of a T cell, a macrophage, a dendritic cell, a monocyte, an NK cell and an NKT cell.

17. The engineered immune cell according to claim 16, **characterized in that** the T cell is a CD4+/CD8+ T cell, a CD4+ helper T cell, a CD8+ T cell, a tumor infiltrating cell, a memory T cell, a naive T cell, a γδ-T cell, or an αβ-T cell.

18. The engineered immune cell according to any one of claims 1-17, **characterized in that** the immune cell is derived from an adult stem cell, an embryonic stem cell, a cord blood stem cell, a progenitor cell, a bone marrow stem cell, an induced pluripotent stem cell, a totipotent stem cell or a hematopoietic stem cell.

19. A nucleic acid molecule, which contains: (i) a nucleic acid sequence encoding a chimeric receptor, and (ii) a nucleic acid sequence encoding CCL3, CCL4 and/or CCL5.

20. The nucleic acid molecule according to claim 19, **characterized in that** the chimeric receptor is a chimeric antigen receptor.

21. The nucleic acid molecule according to claim 19, further comprising a nucleic acid sequence encoding an interleukin.

22. The nucleic acid molecule according to claim 21, **characterized in that** the interleukin is IL-2, IL-7, IL-12, IL-15, IL-21, IL-17, IL-18, IL-23, IL-33, a subunit thereof, or a combination thereof, or a combination of subunits thereof.

23. A vector, containing the nucleic acid molecule according to any one of claims 19-22.

24. A pharmaceutical composition, comprising the engineered immune cell according to any one of claims 1-18, the nucleic acid molecule according to any one of claims 19-22 or the vector according to claim 23, and one or more pharmaceutically acceptable excipients.

25. Use of the engineered immune cell according to any one of claims 1-18, the nucleic acid molecule according to any one of claims 19-22, the vector according to claim 23, or the pharmaceutical composition according to claim 24 in preparation of a medicine for treating cancers, infections or autoimmune diseases.
